# EUROPEAN PATENT APPLICATION

(11) **EP 2 253 272 A1**
(43) Date of publication of application: **24.11.2010**
(21) Application number: 09723275.5
(22) Date of filing: 21.01.2009
(51) Int. Cl.: A61B 5/157, A61B 5/1473, A61B 5/151

(54) **CIRCUIT BOARD FOR COLLECTING BODY FLUID AND BIOSENSOR**

(30) Priority: 21.03.2008 JP 2008073540
(71) Applicant: Nitto Denko Corporation, Ibaraki-shi Osaka 567-8680 (JP)
(72) Inventor: KANETO, Masayuki, Ibaraki-shi Osaka 567-8680 (JP)
(74) Representative: HOFFMANN EITLE
(86) International application number: PCT/JP2009/050817
(87) International publication number: WO 2009/116312

(57) **Abstract**

A circuit board for body fluid collection includes a measurement unit including a puncture needle and an electrode for making contact with the body fluid collected by the puncturing with the puncture needle, wherein the measurement unit is provided in a plural number and disposed radially on a same plane.

## Description

### TECHNICAL FIELD

The present invention relates to a circuit board for body fluid collection, and a biosensor. In particular, the present invention relates to a circuit board for body fluid collection, and a biosensor including the same.

### BACKGROUND ART

Diabetes mellitus includes insulin-dependent (type I) diabetes and non-insulin-dependent (type II) diabetes. The former type of diabetes necessitates regular administration of insulin. Therefore, for a patient with the former type of diabetes, there has been employed a treatment method in which a patient collects his or her blood, measures his or her blood sugar level, and administers to himself or herself insulin at a dosage in accordance with the blood sugar level.
There has been known, for mainly such patients, a blood-sugar-level measuring device which allows a patient to personally collect blood on his/her own and to measure a blood sugar level.

For example, there has been proposed a fluid collecting device including a reaction zone which is provided at the center of a main body and into which electrodes are inserted; a puncture needle outwardly protruding from the center of the main body; and a capillary channel providing communication between the electrodes and the puncture needle (ref: for example, Patent Document 1 shown below).
Patent Document 1: Japanese Unexamined Patent Publication No. 2004-493

### DISCLOSURE OF THE INVENTION

### PROBLEM TO BE SOLVED BY THE INVENTION

In the above-described fluid collecting device of Patent Document 1, the puncture needle and the reaction zone are formed integrally with the main body, and therefore the measurement preparation is easy. However, with this fluid collecting device, the electrode which is a member separate from the reaction zone has to be inserted into the reaction zone to perform a measurement on the blood component. Therefore, there is a disadvantage in that blood detection accuracy becomes unstable and accurate measurement cannot be performed.
Moreover, in type I diabetes, depending on symptoms, the patient has to measure the blood-sugar level several times per day, to be specific, before every meal or after every meal.

However, with the above-described fluid collecting device of Patent Document 1, only one puncture needle is provided in one device, and therefore in order to avoid repetitive use of the puncture needle, the measurement can be performed only once.
Therefore, when the measurement is performed several times as described above with the above-described fluid collecting device, it is necessary that the used fluid collecting device is disposed and a new fluid collecting device is prepared afterwards. Thus, with such a fluid collecting device, the measurement preparation as described above is complicate, and an increase in running costs is inevitable.

An object of the present invention is to provide a circuit board for body fluid collection that is capable of accurate measurement on a body fluid component with a simple structure, and even capable of easy measurement a plurality of times with one circuit board for body fluid collection; and a biosensor including such a circuit board for body fluid collection.

### MEANS FOR SOLVING THE PROBLEM

To achieve the above-described object, a circuit board for body fluid collection of the present invention includes a measurement unit including a puncture needle and an electrode for making contact with the body fluid collected by the puncturing with the puncture needle, wherein the measurement unit is provided in a plural number and disposed radially on a same plane.
The circuit board for body fluid collection includes the measurement unit including the puncture needle and the electrode. Thus, by causing a body fluid to flow out by puncturing with the puncture needle, the body fluid that was caused to flow out is easily brought in contact with the electrode in the measurement unit. As a result, with the circuit board for body fluid collection, a measurement on a component in body fluid can be performed easily with a simple structure.

Moreover, with the circuit board for body fluid collection, a measurement on a component in body fluid can be performed a plurality of times using a plurality of measurement units provided in one circuit board for body fluid collection.
Furthermore, in the circuit board for body fluid collection, the measurement units are disposed radially on the same plane, and therefore after using one measurement unit, the used measurement unit can be changed to an unused measurement unit that is adjacent at an upstream side in a rotational direction to the used measurement unit by rotating the circuit board for body fluid collection in a circumferential direction. Therefore, at every measurement in a plurality of measurements, the measurement unit can be changed easily.

It is preferable that, in the circuit board for body fluid collection of the present invention, the measurement unit includes a bending portion that is bendable at an upstream side of a distal end of the puncture needle in the puncturing direction.
With the circuit board for body fluid collection, when using the measurement unit, by bending the measurement unit at the bending portion, the distal end of a desired puncture needle can be brought away from the plane where the plurality of units are disposed. Thus, with the puncture needle that was bent, reliable puncturing can be performed.

It is preferable that, in the circuit board for body fluid collection of the present invention, an opening is provided at a center of the circuit board for body fluid collection, and an engage portion that can be engaged with a driving member for rotating the circuit board for body fluid collection in a circumferential direction is formed at an inner circumferential surface of the opening of the circuit board for body fluid collection.
With the circuit board for body fluid collection, by engaging the engage portion with the driving member, and driving the driving member, the circuit board for body fluid collection can be rotated reliably in a circumferential direction. Thus, the measurement unit can be changed more easily at every measurement in a plurality of measurements.

A biosensor of the present invention includes the above-described circuit board for body fluid collection, and a determination unit that is electrically connected to the electrode and performs a measurement on a component in body fluid.
With the biosensor, a measurement on a component in body fluid can be easily performed in such a way that the body fluid that was caused to flow out by the above-described circuit board for body fluid collection is brought into contact with the electrode, and then measured with the determination unit that is electrically connected to the electrode.

### EFFECT OF THE INVENTION

With the circuit board for body fluid collection according to the present invention, a measurement on a component in body fluid can be performed a plurality of times with the measurement unit that is provided in a plural number in one circuit board for body fluid collection while an easy measurement on a component in body fluid is achieved with a simple structure. Furthermore, at every measurement in a plurality of measurements, the measurement unit can be easily changed.
Furthermore, with the biosensor according to the present invention, a measurement on a component in body fluid can be easily performed.

### BRIEF DESCRIPTION OF THE DRAWINGS

FIG. 1 illustrates a plan view of a circuit board for blood collection as an embodiment of the circuit board for body fluid collection of the present invention.
FIG. 2 illustrates an enlarged plan view of a measurement unit of the circuit board for blood collection shown in FIG. 1.
FIG. 3 illustrates an enlarged rear view of the measurement unit of the circuit board for blood collection shown in FIG. 1.
FIG. 4 illustrates a cross-sectional view taken along line A-A in FIG. 2.
FIG. 5 illustrates production steps for explaining a method for producing a circuit board for collection: (a) illustrating a step of preparing a metal substrate, (b) illustrating a step of forming an insulating base layer, and (c) illustrating a step of forming a conductive pattern including three electrodes.
FIG. 6 illustrates, production steps subsequent to FIG. 5 for explaining a method for producing a circuit board for collection: (d) illustrating a step of forming an insulating cover layer, (e) illustrating a step of forming a plurality of measurement units including puncture needles, and engage grooves by trimming the metal substrate, and (f) illustrating a step of applying a chemical agent to the electrode.
FIG. 7 illustrates schematic perspective views of a blood-sugar-level measuring device as an embodiment of a biosensor of the present invention, in which the circuit board for blood collection shown in FIG. 1 is mounted.
FIG. 8 illustrates sectional side views for explaining a method for using the blood-sugar-level measuring device shown in FIG. 7.

### EMBODIMENT OF THE INVENTION

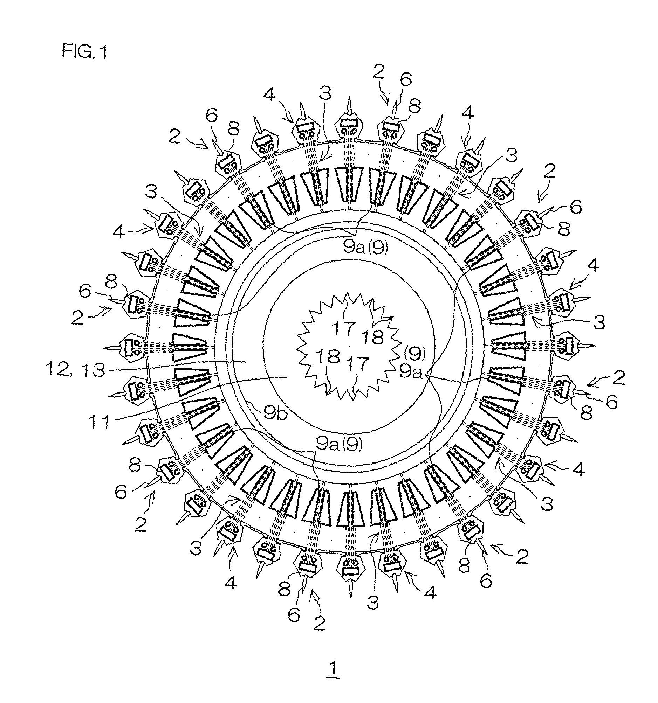

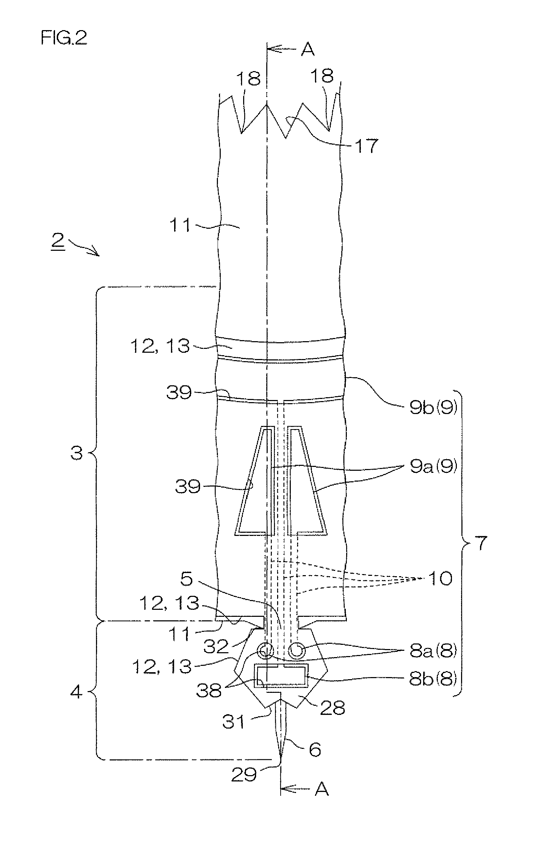

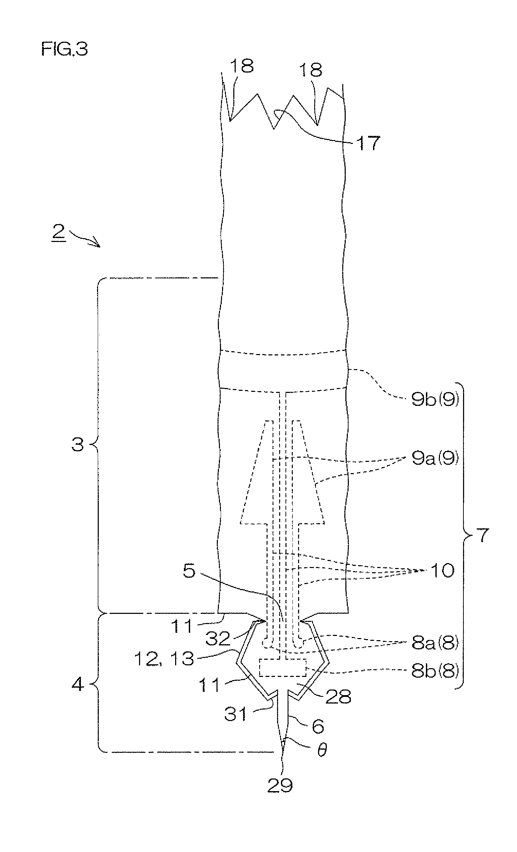

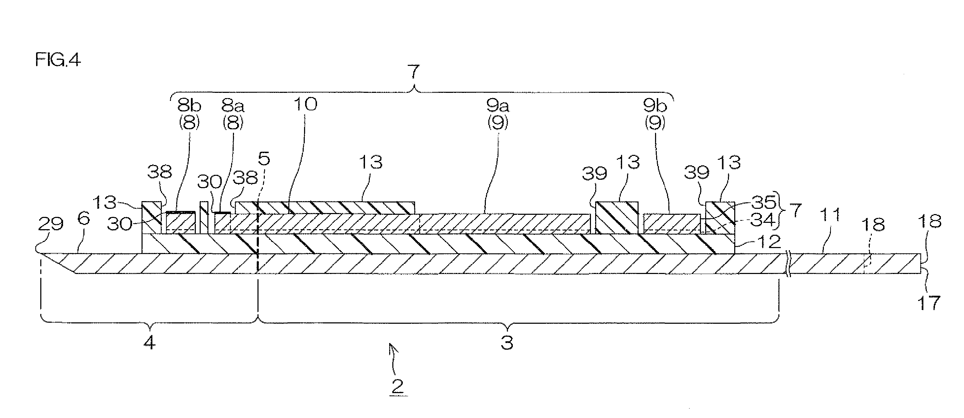

FIG. 1 illustrates a plan view of a circuit board for blood collection as an embodiment of the circuit board for body fluid collection of the present invention; FIG. 2 illustrates an enlarged plan view of a measurement unit of the circuit board for blood collection shown in FIG. 1; FIG. 3 illustrates an enlarged rear view of the measurement unit of the circuit board for blood collection shown in FIG. 1; FIG. 4 illustrates a cross-sectional view taken along line A-A in FIG. 2; and FIGs. 5 and 6 illustrate production steps for explaining a method for producing a circuit board for collection.

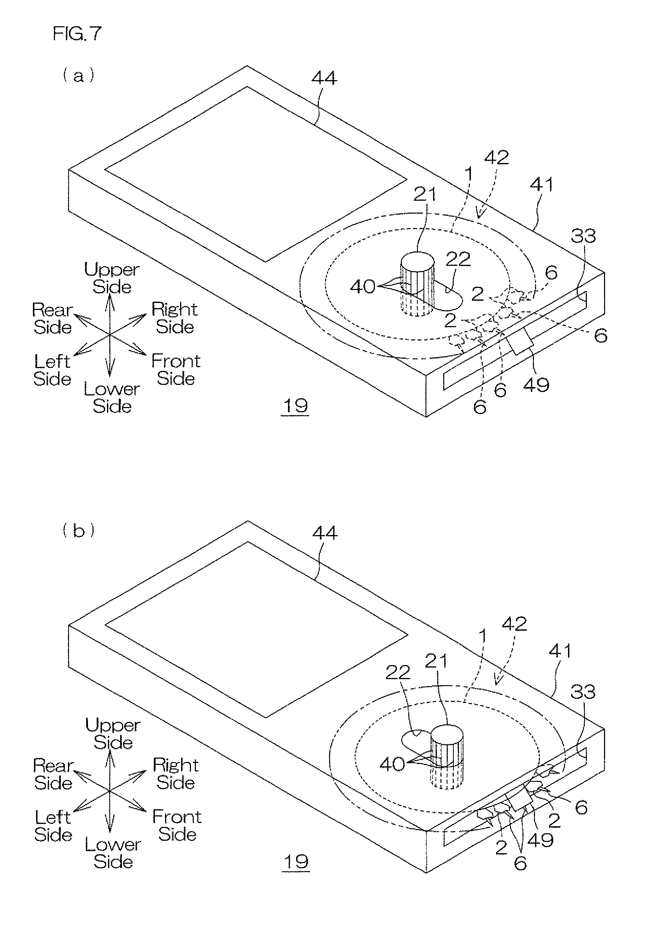

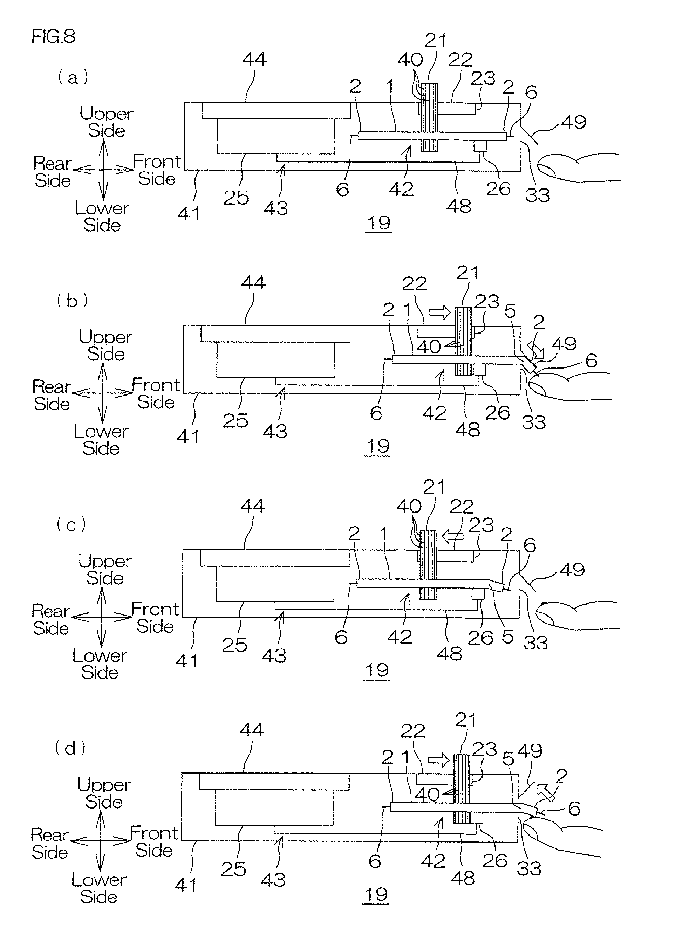

FIG. 1 shows a circuit board for blood collection 1, which is to be mounted in a blood-sugar-level measuring device 19 (ref: FIGs. 7 and 8) to be mentioned later, for a patient to puncture his/her skin of, for example, finger to collect blood, to measure a glucose level in the collected blood. The circuit board for blood collection 1 is prepared as a multiple use (consecutively usable) type, which enables a plurality of measurements.

The circuit board for blood collection 1 is formed into a generally disk shape, with a center opening 17 as an opening formed at a center thereof. The circuit board for blood collection 1 has a plurality (32 units) of measurement units 2 disposed radially in the same plane as that of the circuit board for blood collection 1, at an outer side in the radial direction of the center opening 17. At the inner circumferential surface of the center opening 17 of the circuit board for blood collection 1, engage grooves 18 as an engage portion to be mentioned later are formed.

As shown in FIGs. 2 and 3, the measurement unit 2 is integrally provided with an inner portion 3 disposed at an inner side in the radial direction, and an outer portion 4 disposed at an outer side in the radial direction of the inner portion 3.
The inner portion 3 is disposed so as to be spaced apart from the engage grooves 18 of the circuit board for blood collection 1 in the radial direction, and as shown in FIG. 1, is formed continuously with an inner portion 3 of a measurement unit 2 that is adjacent in a circumferential direction. The outer end face in the radial direction of the inner portion 3 is formed, as shown in FIGs. 2 and 3, into an arc (or generally straight line) shape when viewed from top, and such a shape allows the circuit board for blood collection 1 to be formed into a generally disk shape (or generally regular polygon (generally regular tricontadigon)).

The outer portion 4 is disposed so as to protrude outwardly in the radial direction from generally a center in the circumferential direction of the outer end face in the radial direction of the inner portion 3. The outer portion 4 is formed so that its length in the circumferential direction is shorter than that of the inner portion 3. In this fashion, the outer portion 4 is disposed, as shown in FIG. 1, such that the outer portions 4 of the measurement units 2 that are adjacent in the circumferential direction are disposed with a space therebetween in the circumferential direction. The outer portion 4 is formed, as shown in FIGs. 2 and 3, from an electrode portion 28 that is formed into a generally pentagon shape when viewed from top, and a puncture needle 6 that is disposed at an outer side in the radial direction of the electrode portion 28.

The measurement unit 2 includes one puncture needle 6 and a conductive pattern 7.
The puncture needle 6 is provided to collect blood by puncturing. That is, the puncture needle 6 is disposed, in the outer portion 4, adjacent to and at an outer side (that is, a downstream side in the puncturing direction) in the radial direction of the electrode portion 28, and is formed integrally with the electrode portion 28. To be specific, the puncture needle 6 protrudes outwardly in the radial direction from the center in the circumferential direction of the outer end portion in the radial direction of the electrode portion 28. The puncture needle 6 is formed into a generally triangle (isosceles triangle) shape when viewed from top, and its distal end 29 (outer end portion in the radial direction) is tapered along the radial direction to form an acute angle.

Angle θ (ref: FIG. 3) of the distal end 29 of the puncture needle 6 is, for example, 10 to 30°, or preferably 15 to 25°. When angle θ of the distal end 29 is below 10°, the skin puncturing may not be performed because of insufficient strength. On the other hand, when angle θ exceeds 30°, the puncturing may become difficult. The length of the puncture needle 6 in the radial direction (puncturing direction) is, for example, 0.5 to 10 mm, and the length of the puncture needle 6 in the circumferential direction (width of the inner portion in the radial direction) is, for example, 0.3 to 3 mm. When the length in the puncturing direction and the width of the puncture needle 6 are below the above-described range, the blood collection may become difficult, and when the length in the puncturing direction and the width of the puncture needle 6 exceed the above-described range, damage at the punctured portion may increase.

The conductive pattern 7 includes three electrodes 8, three terminals 9, and three wirings 10.
The three electrodes 8 are provided for making contact with the collected blood by puncturing with the puncture needle 6 to be mentioned later, and are disposed adjacently in the circumferential direction and in the radial direction in the electrode portion 28.
To be more specific, two electrodes 8a among the three electrodes 8 are disposed to face each other with a space therebetween in the circumferential direction in the electrode portion 28. The two electrodes 8a are formed into a generally circular shape when viewed from top.

The remaining one electrode 8b is disposed at an outer side in the radial direction to face the two electrodes 8b with a space therebetween in the electrode portion 28. The electrode 8b is formed into a generally rectangular shape when viewed from top, and extends over the two electrodes 8b.
The three electrodes 8 correspond to a working electrode, a counter electrode, and a reference electrode, respectively. The diameter of the two electrodes 8a is, for example, 100 µm to 2.5 mm, and the length of a side of the one electrode 8b is, for example, 100 µm to 2.5 mm. The three electrodes 8 are disposed, for example, within 0.2 to 5 mm, or preferably 0.5 to 3 mm from the distal end 29 of the puncture needle 6 in the radial direction. When the space between the distal end 29 of the puncture needle 6 and the electrodes 8 is too short, the electrodes 8 sting the skin along with the puncture needle 6, and a chemical agent 30 (described later) applied to the surface of the electrodes 8 may be dispersed into the body, which may hinder accurate measurements. On the other hand, when the space between the distal end 29 of the puncture needle 6 and the electrodes 8 is too long, a structure for utilizing aspiration or capillarity to introduce blood from the puncture needle 6 to the electrodes 8 becomes necessary.

The three terminals 9 are provided in correspondence to the three electrodes 8, and are disposed at the inner portion 3 to be connected to a CPU 25 to be mentioned later.
To be more specific, two terminals 9a correspond to the two electrodes 8a, and are disposed to face each other in the circumferential direction with a space therebetween in the inner portion 3. The two terminals 9a are formed into a generally tapered shape when viewed from top, and the length (width) thereof in the circumferential direction gradually narrow toward an inner side in the radial direction. To be specific, internal end edges that face each other in the circumferential direction of the two terminals 9a are disposed in parallel along the radial direction. The external end edges in the circumferential direction of the two terminals 9a are formed along directions that cross each other in the radial direction.

The remaining one terminal 9b corresponds to the one electrode 8b, and is disposed to face the two terminals 9a with a space therebetween at an inner side in the radial direction. The one terminal 9b is formed into a generally arc flat belt shape in respective measurement units 2 along the circumferential direction. That is, the terminal 9b is formed continuously, as shown in FIG. 1, into a generally loop shape in one circuit board for blood collection 1.
That is, the one terminal 9b is provided in one circuit board for blood collection 1. In this fashion, the one terminal 9b is formed continuously with the terminal 9b of another measurement unit 2 that is adjacent in the circumferential direction, and the plurality of the terminals 9b of the measurement units 2 are used as a one common terminal.

In the three terminals 9, the length of a side of the two terminals 9a is, for example, 0.5 to 10 mm, and the length in the radial direction (width) of the one terminal 9b is, for example, 0.5 to 5 mm.
The three wirings 10 are provided, as shown in FIGs. 2 and 3, so as to run through the inner portion 3 and the outer portion 4, and are disposed in parallel with a space provided therebetween in the circumferential direction. The three wirings 10 are provided along the radial direction so as to electrically connect respective electrodes 8 and terminals 9 corresponding to the electrodes 8. The respective electrodes 8, respective terminals 9, and the wirings 10 that allow connection between them are provided continuously and integrally. The length of the wirings 10 in the circumferential direction is, for example, 0.01 to 2 mm, and the length of the wirings 10 in the radial direction is, for example, 5 to 28 mm.

Each of the measurement units 2 has a bending portion 5 and a stopper portion 31.
The bending portion 5 is provided, as shown in FIG. 8, so as to be bendable at an inner side in the radial direction with respect to the distal end 29 of the puncture needle 6 (an upstream side in the puncturing direction). That is, the bending portion 5 is formed, as shown in FIGs. 2 and 3, as a straight line portion extending along the circumferential direction between the inner portion 3 and the outer portion 4.

The bending portion 5 is formed at an adjacent portion where the inner portion 3 and the outer portion 4 are adjacent to each other, by a cutting portion 32 that is cut finely toward an inner side in the circumferential direction, as an hourglass portion that is narrow in the length in the circumferential direction.
In this fashion, the bending portion 5 is formed as a fragile portion between the inner portion 3 and the outer portion 4, and therefore the bending portion 5 is provided so that the outer portion 4 is bendable with respect to the inner portion 3.
The stopper portion 31 is provided, in the outer portion 4, at an outer end portion in the radial direction of the electrode portion 28, so as to prevent the puncture needle 6 to deeply pierce the skin excessively. To be specific, the stopper portion 31 is formed, in the electrode portion 28, such that the outermost peak in the radial direction of the generally regular pentagon shape when viewed from top is dented inwardly in the radial direction. That is, the stopper portion 31 is provided, in the electrode portion 28, so as to protrude from both outer sides in the circumferential directions (both outer sides in the circumferential directions and lateral sides in a direction that is oblique to the radial direction) with the puncture needle 6 interposed therebetween. The end edge of the stopper portion 31 in the radial direction (downstream side in the puncturing direction) and the distal end 29 of the puncture needle 6 are spaced apart by, for example, 0.3 to 2 mm.

The engage grooves 18 are formed, as shown in FIG. 1, on the entire inner circumferential surface of the center opening 17 of the circuit board for blood collection 1, so as to be engaged with a driving shaft 21 as a driving member to be mentioned later. To be specific, the engage grooves 18 are formed into a spline shape, in which grooves and projections are alternately arranged.
The circuit board for blood collection 1 includes, as shown in FIG. 4, a metal substrate 11, an insulating base layer 12 laminated onto the metal substrate 11, a conductive pattern 7 laminated onto the insulating base layer 12, and an insulating cover layer 13 provided on the insulating base layer 12 to cover the conductive pattern 7.

As shown in FIGs. 1 and 4, the metal substrate 11 is made of metal foil and the like, and formed into a sheet having the outline shape of the circuit board for blood collection 1. That is, the metal substrate 11 is formed, in the circuit board for blood collection 1, as one sheet.
Examples of the metal material that forms the metal substrate 11 include nickel, chromium, iron, and stainless steel (SUS304, SUS430, and SUS316L). Preferably, stainless steel is used. The thickness of the metal substrate 11 is, for example, 10 to 300 µm, or preferably 20 to 100 µm. When the thickness is below 10 µm, the skin puncturing (described later) may not be performed because of insufficient strength. On the other hand, when the thickness exceeds 300 µm, the puncturing may cause pain and damage the skin excessively, and the bending portion 5 may not be bended smoothly.

From the metal substrate 11, the inner portion 3, the outer portion 4 (electrode portion 28 and puncture needle 6), and the engage grooves 18 are formed. Because the puncture needle 6 is formed from the metal substrate 11 made of the above-described metal material, reliable puncturing can be achieved. The engage grooves 18 are formed from the metal substrate 11 I made of the above-described metal materials, and therefore reliable rotation of the circuit board for blood collection 1 can be achieved.

The insulating base layer 12 is formed on the surface of the metal substrate 11 corresponding to the inner portion 3 and the outer portion 4. The insulating base layer 12 is formed, as shown in FIG. 2, so as to expose the outer end portion in the radial direction of the metal substrate 11 when viewed from top in the inner portion 3. The insulating base layer 12 is formed, as shown in FIG. 3, when viewed from top, in the outer portion 4 including the stopper portion 31, to bulge from the peripheral end portion of the metal substrate 11, to be more specific, from the outer end portion in the circumferential direction and both end portions in the radial direction of the metal substrate 11, toward an outer side in the circumferential directions and both radial directions.

Examples of the insulating material that forms the insulating base layer 12 include synthetic resins such as polyimide resin, polycarbonate resin, polyethylene resin, polyethyleneterephthalate resin, epoxy resin, and fluorocarbon resin. In view of mechanical durability, and chemical resistance, preferably, polyimide resin is used. The thickness of the insulating base layer 12 is, for example, 3 to 50 µm, or preferably 5 to 25 µm. When the thickness is below 3 µm, there may be a case where an insulation defect such as pinholes is caused. On the other hand, when the thickness exceeds 50 µm, cutting and trimming may become difficult.

The conductive pattern 7 is formed, as shown in FIG. 4, on the surface of the insulating base layer 12, and is formed as a wiring circuit pattern including the above-described three electrodes 8, three terminals 9, and three wirings 10.
Examples of the conductive material that forms the conductive pattern 7 include metal materials such as iron, nickel, chromium, copper, gold, silver, platinum, and alloys thereof.
The conductive material is selected appropriately in view of adhesiveness to the insulating base layer 12 and the insulating cover layer 13, and easy workability. Two or more conductive materials may be laminated as well. The thickness of the conductive pattern 7 is, for example, 5 to 50 µm, or preferably 10 to 20 µm.

The insulating cover layer 13 is provided on the surface of the insulating base layer 12 so as to cover the wirings 10. The peripheral end portion of the insulating cover layer 13 is, as shown in FIGs. 2 to 4, disposed so as to coincide with the peripheral end portion of the insulating base layer 12 when viewed from top.
The insulating cover layer 13 is formed with, as shown in FIG. 4, electrode-side openings 38 to expose the electrodes 8, and terminal-side openings 39 to expose the terminals 9. To be specific, the electrode-side openings 38 are formed, as shown in FIG. 2, so as to encircle the electrodes 8 and to be slightly larger than the electrodes 8 when viewed from top. The terminal-side openings 39 are formed so as to encircle the terminals 9 and to be slightly larger than the terminals 9 when viewed from top. For the insulating material that forms the insulating cover layer 13, the above-described insulating materials of the insulating base layer 12 are used. The thickness of the insulating cover layer 13 is, for example, 2 to 50 µm.

Then, as shown in FIG. 3, the stopper portion 31 is formed from the bulging portion of the above-described insulating base layer 12 and the insulating cover layer 13. Usually, the insulating material that forms the stopper portion 31 is softer than the metal material that forms the metal substrate 11, and therefore when preventing excessive puncturing with the puncture needle 6, damage to the skin that makes contact with the stopper portion 31 can be effectively prevented.

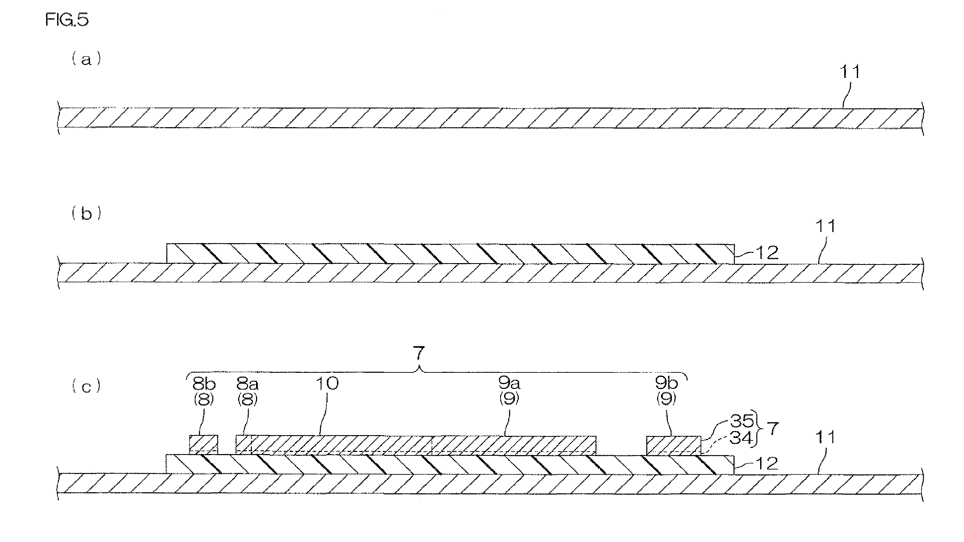

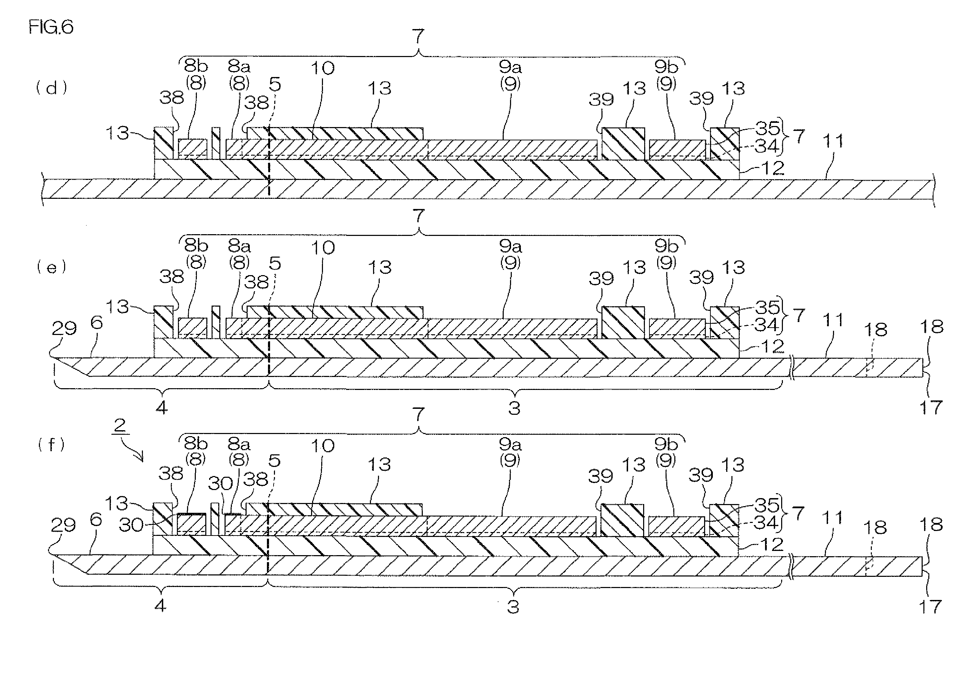

The bending portion 5 is formed from the above-described metal substrate 11, the insulating base layer 12, and the insulating cover layer 13.
Next, with reference to FIGs. 5 and 6, a method for producing the circuit board for blood collection 1 is explained.
In this method, first, as shown in FIG. 5 (a), the metal substrate 11 is prepared. For the metal substrate 11, for example, a metal foil in the form of an elongated sheet which ensures a large number of the metal substrates 11 is prepared. From such an elongated metal foil, a plurality of circuit boards for blood collection 1 are produced by trimming the metal substrates 11 in the subsequent steps.

Next, in this method, as shown in FIG. 5 (b), the insulating base layer 12 is formed on the metal substrate 11. For the formation of the insulating base layer 12, for example, the following methods are used: a method in which a varnish of a photosensitive synthetic resin is applied on the surface of the metal substrate 11, photoprocessed, and then cured; a method in which a synthetic resin film is laminated onto the surface of the metal substrate 11, an etching resist having the same pattern as that of the insulating base layer 12 is laminated onto the surface of the film, and afterwards, the film exposed from the etching resist is wet-etched; a method in which a synthetic resin film that is punched by a machine in advance is laminated onto the surface of the metal substrate 11; and a method in which a synthetic resin film is laminated onto the surface of the metal substrate 11 first, and then subjected to discharge processing or laser processing. In view of processing accuracy, the method in which a varnish of a photosensitive synthetic resin is applied on the surface of the metal substrate 11, photoprocessed, and then cured is preferably used.

Afterwards, in this method, as shown in FIG. 5 (c), the conductive pattern 7 is formed. For the formation of the conductive pattern 7, a known patterning method for forming printed wirings is used, such as an additive method and a subtractive method. In view of achieving a minute pattern, preferably, the additive method is used. In the additive method, for example, a metal thin film 34 (broken line) is formed on the surface of the insulating base layer 12 by chemical vapor deposition or sputtering, and after a plating resist is formed on the surface of the metal thin film 34, a plating layer 35 is formed on the surface of the metal thin film 34 exposing from the plating resist by electrolytic plating using the metal thin film 34 as a seed film.

The conductive pattern 7 can also be formed only of the metal thin film 34 by chemical vapor deposition or sputtering.
Upon formation of the conductive pattern 7, a different type of metal plating layer may also be formed on the surface of the electrodes 8 and the surface of the terminals 9 by further electrolytic plating or electroless plating, although not shown in the drawings. The thickness of the metal plating layer is preferably 0.05 to 20 µm.

Then, in this method, as shown in FIG. 6 (d), the insulating cover layer 13 is formed. For the formation of the insulating cover layer 13, the same method as the method for forming the insulating base layer 12 is used. A preferable method that may be used is the method in which a varnish of a photosensitive synthetic resin is applied on the surface of the insulating base layer 12 so as to cover the conductive pattern 7, photoprocessed, and then cured. When the insulating cover layer 13 is to be formed into a pattern, the electrode-side openings 38 and the terminal-side openings 39 may be formed by forming the insulating cover layer 13 into a pattern having the electrode-side openings 38 and the terminal-side openings 39; and the electrode-side openings 38 and the terminal-side openings 39 may also be formed, for example, by a discharge processing method, and a laser processing method.

Afterwards, as shown in FIG. 6 (e), the metal substrate 11 is trimmed to simultaneously form the inner portion 3; the outer portion 4 (including the stopper portion 31) including the puncture needle 6 and the electrode portion 28; the engage grooves 18; and the bending portion 5. For the trimming of the metal substrate 11, for example, discharge processing, laser processing, mechanical punching processing (for example, punching processing), or etching processing is used. In view of easy cleaning after processing, etching processing (wet etching) is preferably used.

In this manner, the circuit board for blood collection 1 including the plurality of measurement units 2 provided with the puncture needles 6 and the electrodes 8 can be obtained.
In the obtained circuit board for blood collection 1, as shown in FIG. 6(f), the chemical agent 30 is applied on the electrodes 8: that is, for example, glucose oxidase, or glucose dehydrogenase, as an enzyme, and for example, potassium ferricyanide, ferrocene, or benzoquinone as a mediator, alone or in combination is applied. For the application of the chemical agent 30, for example, an appropriate method such as a dipping method, a spray method, or an inkjet method is used.

Depending on the type of the chemical agent 30, it is also possible to, after the plating layer of a different metal is formed on the surface of the electrodes 8 as described above, further form a coating of a different metal in advance, and provide a predetermined potential difference therebetween. To be specific, for example, after a gold plating layer is formed, silver or silver chloride is applied on the surface of the gold plating layer.
FIG. 7 illustrates schematic perspective views of a blood-sugar-level measuring device as an embodiment of a biosensor of the present invention, in which the circuit board for blood collection 1 shown in FIG. 1 is mounted; and FIG. 8 illustrates sectional side views for explaining a method for using the blood-sugar-level measuring device shown in FIG. 7. In FIG. 8, the right side on the plane of the sheet is referred to as "front side", the left side on the plane of the sheet is referred to as "rear side", the upper side on the plane of the sheet is referred to as "upper side", the lower side on the plane of the sheet is referred to as "lower side", the front side on the plane of the sheet is referred to as "left side", and the back side on the plane of the sheet is referred to as "right side"; and directions indicated in FIG. 7 are in accordance with the directions indicated in FIG. 8.

Next, with reference to FIGs. 7 and 8, the method for using the blood-sugar-level measuring device 19 in which the circuit board for blood collection 1 is mounted is explained.
In FIGs. 7 and 8, the circuit board for blood collection 1 obtained as described above is mounted in the blood-sugar-level measuring device 19 and used, as described above, for a patient to puncture his/her skin of, for example, finger to collect blood, to measure a glucose level in the collected blood.

That is, the blood-sugar-level measuring device 19 includes a casing 41, a blood collection unit 42, and a determination unit 43 (omitted in FIG. 7) that measures a glucose level in blood, and a display unit 44.
The casing 41 is prepared to accommodate the members of the blood-sugar-level measuring device 19, and is formed into a box. To be specific, the casing 41 contains the blood collection unit 42 and the determination unit 43; and the display unit 44 is provided on the surface of the casing 41. The casing 41 is formed with a front side opening 33, an upper side opening 22, and a bending guide portion 49.

The front side opening 33 is formed on the front wall of the casing 41 so as to extend in the left and right directions to be formed into a generally rectangular shape when viewed from the front, and to expose some (a few units) of the measurement units 2 when the circuit board for blood collection 1 advances forward, as described later.
The upper side opening 22 is formed at a center in the left-right directions of a front side of the upper wall of the casing 41 as a long hole that extends in front and rear directions. To be specific, the upper side opening 22 is formed so that the driving shaft 21, to be described later, is inserted slidably in front and rear directions.

The bending guide portion 49 is provided at a center in the left and right directions of the front wall of the casing 41 and at an upper end edge of the upper side opening 22 of the front wall, and is formed into a generally flat plate shape. The bending guide portion 49 is provided such that its front end edge is swingable in up and down directions with its back end edge as the supporting point, and is disposed to extend obliquely forward toward a lower side from the front wall of the casing 41 so as to usually block ahead of the upper side opening 22. The bending guide portion 49 closes, when puncturing with the circuit board for blood collection 1, so as to block ahead of the upper side opening 22 (ref: FIG. 8 (b)), while when measuring a blood-sugar level, the bending guide portion 49 opens, so as to expose the upper side opening 22 and then to expose the foremost measurement unit 2 therefrom, and so as not to make contact with the foremost measurement unit 2 (ref: FIG. 8 (d)).

When puncturing with the circuit board for blood collection 1, an angle between the direction along the bending guide portion 49 and the front and rear directions is set to, when the bending guide portion 49 is viewed from a side, for example, 15 to 60°, or preferably 20 to 45°.
The blood collection unit 42 includes, as shown in FIG. 8, the driving shaft 21, a guide portion 23, and the circuit board for blood collection 1.

The driving shaft 21 is disposed such that its axis extends along up and down directions, and on its entire outer circumferential surface, driving grooves 40 are provided so as to be engaged with the engage grooves 1 of the circuit board for blood collection 1. That is, the driving shaft 21 is inserted into the center opening 17 (ref: FIG. 1) of the circuit board for blood collection 1, and the driving grooves 40 are engaged with the engage grooves 18 (ref: FIG. 1). In this way, the driving shaft 21 is engaged with the engage grooves 18 in a manner such that the driving shaft 21 is removable along up and down directions but not rotatable relative to the engage grooves 18, and is provided so as to allow the circuit board for blood collection 1 to rotate in the circumferential direction with the axis of the driving shaft 21 as the center.

The guide portion 23 is provided at a peripheral end of the upper side opening 22 of the upper wall of the casing 41. To be specific, the guide portion 23 is provided so as to guide advancing and retreating of the driving shaft 21 in front and rear directions.
The circuit board for blood collection 1 is provided so as to be capable of advancing and retreating in front and rear directions and rotatable in the circumferential direction with the axis of the driving shaft 21 as the center by the driving shaft 21. The circuit board for blood collection 1 is disposed such that the electrodes 8 and the terminals 9 are exposed toward a lower side. When the circuit board for blood collection 1 advances, a few measurement units 2 at the front side expose themselves, and among them, the puncture needle 6 of the measurement unit 2 at the foremost side is brought into contact with the bending guide portion 49.

The determination unit 43 is electrically connected to the electrodes 8, and includes a contact portion 26, and a CPU 25.
The contact portion 26 is provided slidably with respect to the terminals 9 so that when the circuit board for blood collection 1 performs a measurement, the contact portion 26 is brought into contact with the terminals 9 (ref: FIGs. 2 and 3) of the measurement unit 2 that performs the measurement. The contact portion 26 is provided so as to be capable of applying a voltage to the electrodes 8 via the terminals 9, as well as capable of detecting a change in a resistance value between the electrodes 8 when the voltage is applied.

The CPU 25 is electrically connected to the contact portion 26 via a signal wiring 48, and is also connected to the display unit 44. The CPU 25 is provided so as to be capable of calculating a glucose level as a blood-sugar level based on the change in the resistance value between the electrodes 8 detected at the contact portion 26 when the circuit board for blood collection 1 performs a measurement.
The display unit 44 is provided at a rear side of the upper wall of the casing 41; includes, for example, LED; and displays the blood-sugar level measured by the CPU 25.

When using the blood-sugar-level measuring device 19, first, as shown in FIG. 7 (a) and FIG. 8 (a), the driving shaft 21 is slid toward a rear side so that the blood-sugar-level measuring device 19 is ready, and a finger of a patient himself/herself is brought to a lower side of the bending guide portion 49. When the driving shaft 21 has been slid to a rear side in advance, there is no need to slide the driving shaft 21.
In this case, in the blood-sugar-level measuring device 19, all of the measurement units 2 of the circuit board for blood collection 1 are accommodated in the casing 41 without being exposed from the front side opening 33.

In this method, next, as shown in FIG. 7 (b) and FIG. 8 (b), the driving shaft 21 is slid toward a front side to expose the puncture needle 6 from the front side opening 33, and a patient himself/herself punctures his/her finger with the puncture needle 6.
At this time, the circuit board for blood collection 1 is allowed to advance toward a front side to expose a few measurement units 2 out of the measurement units 2 from the front side opening 33, and to bring the measurement unit 2 of the foremost side in contact with the bending guide portion 49, which causes the outer portion 4 to be bent toward an obliquely lower side with respect to the inner portion 3 at the bending portion 5. Then, the puncture needle 6 of the outer portion 4 that was bent is used to puncture the finger.

Because the bending guide portion 49 is disposed at the above-described predetermined angle when viewed from a side, the bending angle of the bending portion 5 is, for example, 15 to 60°, or preferably 20 to 45°.
At this time, upon puncturing with the puncture needle 6, when the stopper portion 31 abuts on the skin, further puncturing is restricted. Thus, the puncturing depth of the puncture needle 6 is, for example, 0.5 to 1.5 mm.

Then, in this method, as shown in FIG. 8 (c), the driving shaft 21 is slid to a rear side, and the puncture needle 6 is drawn out from, for example, a finger, causing a trace amount of bleeding from the punctured portion.
At this time, the measurement unit 2 that was bent by the bending guide portion 49 is brought away from the bending guide portion 49, modifying the bending angle. To be specific, the bending angle at the bending portion 5 is, for example, 15 to 60°, or preferably 30 to 45°.

The bleeding in a trace amount at the punctured portion can be accelerated as necessary by pressing (stressing) in the periphery of the punctured portion.
Next, in this method, as shown in FIG. 8 (d), the front end portion of the bending guide portion 49 is swung upward to open, and the driving shaft 21 is slid again toward a front side to expose the electrodes 8 of the measurement unit 2 at the foremost side from the front side opening 33, so as to bring the electrodes 8 in contact with the punctured portion.

Then, the surface of the electrodes 8 is brought in contact with the blood collected by the puncturing with the puncture needle 6, and the blood is reacted with the chemical agent 30. At this time, the contact portion 26 is brought into contact with the terminals 9, and at the same time, a voltage is applied to the electrodes 8 from the contact portion 26 via the terminal 9. Then, a change in the resistance value between the electrodes 8 at the time of the voltage application is detected by the contact portion 26, and based on the change in the resistance value detected by the contact portion 26, the CPU 25 calculates a glucose level as a blood-sugar level. Then, the blood-sugar level measured by the CPU 25 is displayed at the display unit 44.

Afterwards, in this method, although not shown, by rotating the driving shaft 21 in the circumferential direction with the axis of the driving shaft 21 as the center to rotate the circuit board for blood collection 1, an unused measurement unit 2 that is disposed upstream of and adjacent to the used measurement unit 2 in the rotational direction is disposed at the foremost side. Afterwards, the steps shown in the above-described FIG. 8 (a) to FIG. 8 (d) are performed several times, thereby measuring the blood-sugar level several times.

Then, with the circuit board for blood collection 1 and the blood-sugar-level measuring device 19 including the circuit board for blood collection 1, by causing bleeding by puncturing with the puncture needle 6, and allowing the electrodes 8 of the measurement unit 2 to be brought into contact with the blood that was caused to bleed, a blood-sugar level can be simply measured by the CPU 25 that is electrically connected to the electrodes 8.
As a result, the circuit board for blood collection 1 and the blood-sugar-level measuring device 19 are capable of simply measuring a blood-sugar level with a simple structure.

Furthermore, with the circuit board for blood collection 1, based on the plurality of measurement units 2 provided in one circuit board for blood collection 1, multiple measurements of a blood-sugar level can be achieved.
Furthermore, with the circuit board for blood collection 1, because the measurement unit 2 is radially disposed on the same plane, after using one measurement unit 2, by rotating the circuit board for blood collection 1 in the circumferential direction, the used measurement unit 2 can be changed to an unused measurement unit 2 that is upstream of and adjacent to the used measurement unit 2 in the rotational direction. Therefore, for every measurement in the multiple measurements, the measurement unit 2 can be changed easily.

Furthermore, with the circuit board for blood collection 1, upon using the measurement unit 2, by bending the measurement unit 2 at the bending portion 5, the distal end 29 of the desired puncture needle 6 can be brought away from the plane where the plurality of measurement units 2 are disposed. Therefore, reliable puncturing can be performed by the puncture needle 6 that was bent.
Furthermore, with the circuit board for blood collection 1, by engaging the engage grooves 18 with the driving shaft 21, and rotating the driving shaft 21 in the circumferential direction, the circuit board for blood collection 1 can be reliably rotated in the circumferential direction. Therefore, at every measurement in the plurality of measurements, the measurement unit 2 can be changed more easily.

Although 32 units of the measurement unit 2 are provided in the circuit board for blood collection 1 in the above description referring to FIG. 1, the number of the unit is not particularly limited, and is selected appropriately in accordance with the size of, for example, the casing 41, and can be provided in a number of, for example, 10 or more, or preferably 20 or more, and usually 100 or less.
Also, in the above description with reference to FIG. 1, the size of the circuit board for blood collection 1 is appropriately selected in accordance with the size of the casing 41 and the number of the measurement unit 2, and without limitation, the diameter thereof is, for example, 20 mm or more, or for example, 100 mm or less. When the diameter is below the above-described range, the number of the measurement unit 2 may become excessively small. When the diameter exceeds the above-described range, the casing 41 may become excessively large, and handling of the blood-sugar-level measuring device 19 may become difficult.

Although the bending portion 5 is provided between the inner portion 3 and the outer portion 4 in the above description with reference to FIGs. 2 and 3, the bending portion 5 may also be provided, for example, at the puncture needle 6 in the inner portion 3, to be specific, at an inner side portion in the radial direction relative to the distal end 29 of the puncture needle 6 (at an upstream side in the puncturing direction) of the puncture needle 6, that is, in the middle in the radial direction, or an inner edge portion in the radial direction of the puncture needle 6, although not shown.
Although the engage grooves 18 are provided on the entire inner circumferential surface at the center opening 17 of the circuit board for blood collection 1 in the above description with reference to FIG. 1, for example, the engage grooves 18 may be provided only at a portion of the inner circumferential surface, and the remaining surface may be formed into a cylindrical shape (without projections and recesses), although not shown. In such a case, the driving grooves 40 (ref: FIGs. 7 and 8) of the driving shaft 21 are formed into a shape that corresponds to the above-described engage grooves 18.

Furthermore, the engage grooves 18 may be formed as keyways that fit the driving shaft 21 formed into a key.
In the above description, the circuit board for blood collection 1 and the blood-sugar-level measuring device 19 including the circuit board for blood collection 1 are given as examples of the circuit board for body fluid collection of the present invention, and the biosensor including the circuit board for body fluid collection. That is, in the above description, blood is given as an example of the body fluid collected by puncturing with the puncture needle of the circuit board for body fluid collection.

However, the body fluid is not particularly limited as long as it is a liquid in a living body, and examples thereof include extracellular fluid and intracellular fluid. Examples of the extracellular fluid include, other than blood mentioned above, a blood plasma; an intercellular fluid; a lymph fluid; moistures in dense connective tissue, bone, and cartilage; and a transcellular fluid. A measurement on a specific component of the above-described body fluid can be performed with the circuit board for body fluid collection and the biosensor including the circuit board for body fluid collection.

### EXAMPLES

### EXAMPLE 1

### (Production of Circuit Board for Blood Collection Shown in FIG. 1)

First, an elongated sheet metal substrate made of SUS430 and having a thickness of 50 µm was prepared (ref: FIG. 5 (a)).
Then, on the surface of the metal substrate, a varnish of a photosensitive polyimide resin precursor (photosensitive polyamic acid resin) was applied, and dried by heating to form a coating. The coating was then exposed to light, and developed to be formed into a pattern. Thereafter, the coating was heated in a nitrogen atmosphere to 400°C, to form an insulating base layer having a thickness of 10 µm in the abovementioned pattern (ref: FIG. 5 (b)).

Then, on the surface of the insulating base layer, metal thin films formed of a chromium thin film and a copper thin film were formed sequentially by sputtering. Subsequently, a dry film resist was laminated on the surface of the metal thin film, exposed to light, and developed to form a plating resist in a pattern. Then, a plating layer formed of copper was formed on the surface of the metal thin film exposed from the plating resist using the metal thin film as a seed film by electrolytic copper plating, thereby forming a conductive pattern including electrodes, terminals, and wirings (ref: FIG. 5 (c)). Afterwards, the plating resist and the portion of the metal thin film where the plating resist was formed was removed by etching.

The thickness of the conductive pattern was 12 µm, the diameter of the two electrodes (8a) was 0.3 mm, and the length of the long side of the one electrode (8b) was 1.0 mm, and the short side thereof was 0.6 mm. In the two terminals (9a), the length of the internal (in the circumferential direction) end edge was 4 mm; the length of the external (in the radial direction) end edge (long side) along the circumferential direction was 1 mm; and the length of the internal (in the radial direction) end edge (short side) along the circumferential direction was 0.5 mm. The width of the one terminal (9b) was 1 mm. The width of the wiring was 100 µm; the length of the wirings that connect the two electrodes 8a and the two terminals 9a was 25 mm; and the length of the wiring that connects the one electrode 8b and the one terminal 9b was 10 mm.

Afterwards, a varnish of a photosensitive polyimide resin precursor (photosensitive polyamic acid resin) was applied on the surface of the insulating base layer so as to cover the conductive pattern, and dried by heating to form a coating. The coating was then exposed to light, and developed to be formed into a pattern. Afterwards, the coating was heated in a nitrogen atmosphere to 400°C to form an insulating cover layer having a thickness of 5 µm (ref: FIG. 6 (d)). The insulating cover layer was formed such that by forming the electrode-side openings and terminal-side openings, the electrodes and the terminals were exposed but the wirings were covered.

Thereafter, an electrolytic nickel plating layer (thickness 0.5 µm), and an electrolytic gold plating layer (thickness 2.5 µm) were sequentially formed on the surface of the electrodes and the terminals.
Then, a dry film resist was laminated on the surface of the metal substrate, exposed to light, and developed to form an etching resist in a pattern. Then, the metal substrate exposed from the etching resist was etched by wet etching using ferric chloride as an etchant to be trimmed in the above-described pattern having a plurality of measurement units (32 units) having puncture needles (ref: FIG. 6 (e)). By such trimming of the metal substrate, the inner portion, the outer portion (including the stopper portion), the engage grooves, and the bending portion were formed.

The length from the distal end of the puncture needle to the one electrode (8b) (the electrode nearest from the distal end) was 0.5 mm, the angle of the distal end of the puncture needle was 20°, the width of the expanding portion of the stopper portion was 0.5 mm, and the spaced apart-length from the end edge (in the radial direction) of the stopper portion (downstream side in the puncturing direction) to the distal end of the puncture needle was 1.5 mm.
The circuit board for blood collection was thus obtained.

Afterwards, in the obtained circuit board for blood collection, a chemical agent containing glucose oxidase and a potassium ferricyanide solution was applied on the electrode in respective measurement units by inkjet (ref: FIG. 6 (f)).

### (Production of Blood-Sugar-Level Measuring Device Shown in FIGs. 7 and 8)

The obtained circuit board for blood collection was mounted, along with the driving shaft and the guide, in the casing having the display unit (ref: FIGs. 7 and 8).

To mount the circuit board for blood collection, the driving shaft was inserted in the center opening, thus engaging the driving grooves with the engage grooves, and inserting the driving shaft in the guide portion so as to be slidable.

### (Blood-Sugar Level Measurement with Blood-Sugar-Level Measuring Device)

First, the above-described blood-sugar-level measuring device was prepared, and then the finger of a patient was disposed below the bending guide portion (ref: FIG. 7 (a) and FIG. 8 (a)).

Then, the driving shaft was slid toward a front side, and the puncture needle was exposed from the front side opening, thus allowing the patient to puncture his/her finger with the puncture needle 6 (ref: FIG. 7 (b) and FIG. 8 (b)). At this time, among the measurement units, a few measurement units were exposed from the front side opening, and the measurement unit at the foremost side was brought in contact with the bending guide portion, thus bending the outer portion at 45° with respect to the inner portion at the bending portion. Then, the puncture needle of the outer portion that was bent was used to puncture the finger.

Next, the driving shaft was slid toward a rear side, and the puncture needle was withdrawn from the finger, thus causing a trace amount of bleeding at the punctured portion (ref: FIG. 8 (c)). The measurement unit that was bent at the bending portion was thus brought away from the bending guide portion, forming a bending angle at the bending portion of 35°, and modifying the bending angle.
Next, the bending guide portion was opened, and the driving shaft was again slid toward a front side, and the electrodes of the measurement unit at the foremost side were exposed from the front side opening, thus bringing the electrodes near to and in contact with the punctured portion (ref: FIG. 8 (d)).

Then, glucose was oxidized by the blood, and ferricyanide ions reacted. At the same time, a voltage was applied from the contact portion to the electrodes via the terminals. Then, a change in the resistance value between the electrodes at the time of the voltage application was detected by the contact portion, and the CPU calculated a glucose level as a blood-sugar level based on the change in the resistance value. Then, the blood-sugar level measured by the CPU was displayed at the display unit.
Afterwards, in this method, by rotating the driving shaft in the circumferential direction with the axis of the driving shaft as the center, the circuit board for blood collection was rotated, thus disposing an unused measurement unit that was disposed adjacent (upstream side in a rotational direction) to the used measurement unit at the foremost side. Thereafter, the above-described steps were performed, thus measuring a blood-sugar level a plurality of times (32 times in total).

While the illustrative embodiments of the present invention are provided in the above description, such is for illustrative purpose only and it is not to be construed as limiting the scope of the present invention. Modification and variation of the present invention that will be obvious to those skilled in the art is to be covered by the following claims.

### Industrial Applicability

A circuit board for body fluid collection, and a biosensor of the present invention are suitably used, for example, in the field where a blood-sugar level in blood is measured.

## Claims

1. A circuit board for body fluid collection comprising a measurement unit including a puncture needle and an electrode for making contact with the body fluid collected by the puncturing with the puncture needle,
wherein the measurement unit is provided in a plural number and disposed radially on a same plane.

2. The circuit board for body fluid collection according to Claim 1, wherein the measurement unit comprises a bending portion that is bendable at an upstream side of a distal end of the puncture needle in the puncturing direction.

3. The circuit board for body fluid collection according to Claim 1, wherein an opening is provided at a center of the circuit board for body fluid collection, and
an engage portion that can be engaged with a driving member for rotating the circuit board for body fluid collection in a circumferential direction is formed at an inner circumferential surface of the opening of the circuit board for body fluid collection.

4. A biosensor comprising the circuit board for body fluid collection according to Claim 1, and
a determination unit that is electrically connected to the electrode and performs a measurement on a component in body fluid.
